# EUROPÄISCHE PATENTSCHRIFT

(11) **EP 2 674 281 B1**
(45) Veröffentlichungstag und Bekanntmachung des Hinweises auf die Patenterteilung: **21.02.2018**
(21) Anmeldenummer: 12172261.5
(22) Anmeldetag: 15.06.2012
(51) Int. Cl.: B01L 3/00, C12M 1/22, B29C 65/50, B29C 65/00, B29L 31/00

(54) **Probenkammer und Verfahren zum Herstellen einer Probenkammer**
Sample chamber and method for manufacturing a sample chamber
Chambre d'analyse et procédé de fabrication d'une chambre d'analyse

(43) Veröffentlichungstag der Anmeldung: 18.12.2013
(73) Patentinhaber: ibidi GmbH, 82152 Martinsried (DE)
(72) Erfinder: Kahl, Valentin, 82152 Martinsried (DE); Horn, Elias, 81377 München (DE)
(74) Vertreter: Grünecker Patent- und Rechtsanwälte PartG mbB

(56) Entgegenhaltungen:
- DE-A1- 10 159 091
- US-A- 5 759 494
- US-A1- 2003 021 457
- US-A1- 2009 191 621
- US-A1- 2010 136 671

## Beschreibung

Die vorliegende Erfindung betrifft ein Verfahren zum Herstellen einer Probenkammer sowie eine gemäß diesem Verfahren hergestellte Probenkammer.

Insbesondere im Bereich der Zellmikroskopie sind unterschiedlichste Formen von Probenkammern bekannt. Fast alle Probenkammern weisen dabei Strukturen zum Aufnehmen einer Probe, beispielsweise in Form von Mikrofluidkanälen oder Reservoiren, auf. Beispiele für solche Probenkammern sind in der EP 1 886 792 A2, der WO 2008/149914 A2, der WO 2005/079985 oder der DE 101 48 210 gezeigt. Einfache und sehr bekannte Formen umfassen Petrischalen und Multiwellplatten, wie in der DIN EN ISO 24998 oder der ANSI/SBS 2-2004 für Microplates besch rieben.

Mögliche Einsatzgebiete für solche Probenkammern liegen insbesondere im Bereich der Mikroskopie von Molekülen oder Zellen. Die zu untersuchenden Proben werden dabei mit einer Flüssigkeit in ein Reservoir der Probenkammer gegeben und können dann mit hochauflösenden Verfahren (beispielsweise Durchlichtmikroskopie, Fluoreszenzmikroskopie, Konfokalmikroskopie, etc.) untersucht werden.

Bekannte Probenkammern werden typischerweise einkomponentig oder mehrkomponentig hergestellt. Beispiele für einkomponentige Probenkammern sind Glaspetrischalen und Multiwellplatten aus Polystyrol (PS), welche im Spritzgussverfahren hergestellt werden. Mehrkomponentige Probenkammern umfassen üblicherweise wenigstens eine Seitenwandung und eine Bodenplatte, welche durch verschiedene Methoden miteinander verbunden werden. Bekannt sind vor allem Ultraschallverschweißen oder Verkleben. Eine Art der Verbindung durch adhäsive beziehungsweise klebrige Schichten ist beispielsweise in der EP 1 886 792 A2 beschrieben.

Fertigungstechnisch ist es häufig vorteilhaft, die Probenkammern aus mehreren Komponenten oder Bauteilen herzustellen. Ein Verbinden mit Ultraschallverschweißen ist jedoch energieaufwändig. Die Verwendung von adhäsiven beziehungsweise klebrigen Schichten führt dagegen häufig zum Einsatz von Lösungsmitteln. Diese haben jedoch den Nachteil, dass Rückstände bei der Verwendung des Probenträgers zu einer Kontaminierung der Probe führen können.

Aus der US 2003/0021457 A1 ist eine Probenkammer bekannt, die durch Verbinden eines PDMS-Elements mit einem Glasträger hergestellt wird. Zum Verbinden der Teile werden die Kontaktflächen mit einem Plasma behandelt.

Die DE 101 59 091 A1 offenbart ein Verfahren zur Herstellung eines Trägersystems, insbesondere einer Titerplatte oder eines Mikrosystem-Chips, mit mindestens einer Basis- und Bodenplatte, wobei die Basisplatte Öffnungen aufweist und zumindest teilweise mit einem abbindbaren Klebemittel benetzt wird, die Basisplatte mit einer Bodenplatte in Kontakt gebracht wird, und beim Abbinden mindestens eine im Klebemittel enthaltene Klebesubstanz angereichert wird und (i) eine Klebeverbindung zwischen Bodenplatte und Basisplatte und (ii) eine Beschichtung der Innenwände der Vertiefungen der Basisplatte ausbildet wird. Für die kombinierte Klebeschicht/Beschichtungsschicht kommen Elastomere in Betracht.

Elastomer kann dazu neigen, Flüssigkeiten aufzunehmen oder Komponenten in eine Flüssigkeit abzugeben. Es kann also ein Kontaminierungsrisiko entstehen.

Daher ist es Aufgabe der vorliegenden Erfindung, ein Verfahren zum Herstellen einer verbesserten Probenkammer bereitzustellen.

Diese Aufgabe wird durch ein Verfahren gemäß Anspruch 1 gelöst. Vorteilhafte Weiterbildungen der Erfindung sind in den Unteransprüchen angegeben.

Die Erfinder der vorliegenden Anmeldung haben festgestellt, dass durch das Behandeln einer Kontaktfläche, insbesondere die ein Elastomer umfasst, mit einem Plasma, mit Plasmaabgas und/oder mit einem reaktiven Gas eine Oberfläche bereitgestellt werden kann, die eine dauerhafte, lösungsmittelfreie und flüssigkeitsdichte Verbindung von Bauteilen ermöglicht. Mit anderen Worten ist dadurch eine Verbindung ohne Klebstoff und ohne adhäsive Schicht möglich.

Durch das Verbinden der Kontaktfläche des ersten Bauteils mit einer Kontaktfläche des zweiten Bauteils kann insbesondere eine flüssigkeitsdichte Verbindung zwischen den Bauteilen hergestellt werden. Dadurch kann die Probenkammer für Untersuchungen mit Flüssigkeiten verwendet werden.

Dadurch, dass das erste Element das zweite Element zum Reservoir hin wenigstens teilweise verdeckt oder abdeckt kann das Kontaminationsrisiko im Reservoir verringert werden.

Durch das Verbinden der Kontaktfläche des ersten Bauteils mit der Kontaktfläche des zweiten Bauteils können die Bauteile dauerhaft verbunden werden. Mit anderen Worten können die Bauteile dadurch nicht lösbar beziehungsweise nicht zerstörungsfrei lösbar verbunden werden. Dadurch kann also eine besonders stabile Probenkammer erhalten werden.

Als Kontaktfläche des ersten Bauteils wird hierin eine Oberfläche des ersten Bauteils verstanden, die nach dem Verbinden der Bauteile mit einer Oberfläche des zweiten Bauteils in Kontakt oder Berührung steht. Entsprechend wird eine Kontaktfläche des zweiten Bauteils als eine Oberfläche des zweiten Bauteils verstanden, die nach dem Verbinden der Bauteile mit der entsprechenden Oberfläche des ersten Bauteils in Kontakt oder Berührung steht.

Das Plasma kann insbesondere ein Atmosphärenplasma oder eine Korona sein. Dabei wird unter einem Atmosphärenplasma ein Plasma verstanden, das nicht im Unterdruck im Verhältnis zum Atmosphärendruck gezündet wird. Insbesondere kann es sich um ein Atmosphärenplasma mit Stickstoff als Wirkgas handeln. Im Falle einer so genannten Korona kann es sich insbesondere um eine Koronabehandlung mit Umgebungsluft als Wirkgas handeln.

Als Abgas eines Plasmas oder Plasmaabgas können ein oder mehrere ionisierte/aktivierte Gase bezeichnet werden, die durch ein gezündetes Plasma ionisiert beziehungsweise aktiviert wurden.

Als reaktives Gas kann Gas bezeichnet werden, das eine hohe Reaktionsfreudigkeit aufweist, also eine hohe Fähigkeit eine chemische Reaktion einzugehen. Bei dem reaktiven Gas kann es sich beispielsweise um Ozon oder Stickoxide handeln, oder um reaktive Gase die als molekulare Bestandteile Sauerstoff, Stickstoff und/oder Kohlenstoff enthalten.

Unter einem Elastomer versteht man einen elastisch verformbaren Kunststoff. Bei dem Elastomer kann es sich insbesondere um ein Silikon, insbesondere Polydimethylsiloxan (PDMS) handeln. Das Elastomer kann auch ein auf Elastomer basiertes Spritzguss-Silikon sein.

Das Elastomer kann eine Shore-A Härte von mehr als 40, insbesondere von 60 bis 80, aufweisen. Dadurch kann die Stabilität verbessert werden, insbesondere beim Greifen mit einer Hand.

Das erste Bauteil kann auch aus einem Elastomer bestehen. Damit ist eine besonders einfache Herstellung der Probenkammer möglich.

Das zweite Bauteil kann ein Glas umfassen oder aus Glas bestehen. Das Glas kann dabei insbesondere Borosilikatglas, Quarzglas oder Kalk-Natron Glas sein. Auch Kombinationen unterschiedlicher Gläser sind denkbar.

Das zweite Bauteil kann insbesondere mit einem Glas beschichtet sein. Insbesondere kann das zweite Bauteil nur im Bereich der Kontaktfläche des zweiten Bauteils mit Glas beschichtet sein.

Das zweite Bauteil kann einen Kunststoff umfassen. Bei dem Kunststoff kann es sich insbesondere um COC (Cyclo-Olefin-Copolymer), COP (Cyclo-Olefin-Polymer), PE (Polyethylen), PS (Polystyrol), PC (Polycarbonat) und/oder PMMA (Polymethylmetacrylat) handeln. In diesem Fall kann das Verfahren ein Bedampfen des zweiten Bauteils mit einer Glasschicht umfassen, insbesondere mit SiOₓ, insbesondere derart, dass die Kontaktfläche des zweiten Bauteils ein Glas umfasst oder aus Glas besteht.

Das zweite Bauteil kann insbesondere eine Kunststofffolie sein. Unter einer Kunststofffolie kann ein flächiges Element aus Kunststoff verstanden werden, dessen Längsausdehnungen deutlich größer sind als dessen Dicke.

Alternativ oder zusätzlich kann das zweite Bauteil einen Elastomer, insbesondere ein Silikon, oder ein Metall umfassen.

Unabhängig vom Grundmaterial des zweiten Bauteils kann das Verfahren ein Bedampfen des zweiten Bauteils mit einer Glasschicht umfassen, insbesondere mit SiOₓ, insbesondere derart, dass die Kontaktfläche des zweiten Bauteils ein Glas umfasst oder aus Glas besteht.

Die Erfinder der vorliegenden Anmeldung haben gefunden, dass insbesondere zwischen einem mit Plasma, mit Plasmaabgas und/oder einem reaktiven Gas behandelten Elastomer und einem Glas eine besonders vorteilhafte Verbindung erzielt werden kann.

Außerdem hat sich überraschenderweise gezeigt, dass auch im Falle, dass nur das Glas mit Plasma, mit Plasmaabgas und/oder einem reaktiven Gas behandelt wird, eine ausreichende Verbindung erzielt werden kann.

Das erste Bauteil kann gemäß eines nicht unter die Erfindung fallenden Beispiels bezüglich einer zur Kontaktfläche des ersten Bauteils parallelen Symmetrieebene spiegelsymmetrisch ausgebildet sein. Dadurch kann die Herstellung der Probenkammer weiter vereinfacht werden, da während des Produktionsprozesses zwei gleichwertige Ausrichtungen des ersten Bauteils zur Verfügung stehen.

In diesem Fall kann das erste Bauteil insbesondere aus einem Elastomer bestehen.

Das erste Bauteil kann außerdem einen thermoplastischen Kunststoff umfassen. Das erste Bauteil kann insbesondere ein Verbund aus einem thermoplastischen Kunststoff und einem Elastomer sein. Ein Verbund aus einem thermoplastischen Kunststoff (Thermoplast) und einem Elastomer kann sich positiv auf die Stabilität auswirken. Insbesondere kann der Thermoplast die notwendige Stabilität erzeugen, während das Elastomer interne mechanische Spannungen ausgleichen kann. Zu solchen mechanischen Spannungen kann es insbesondere durch thermische Beanspruchung, zum Beispiel bei einer Dampfsterilisation (üblicherweise bei etwa 121°C) kommen. Außerdem kann durch den Thermoplast die Stabilität der Probenkammer beim Greifen mit der Hand verbessert werden.

Bei dem Thermoplast oder thermoplastischen Kunststoff kann es sich insbesondere um COC, COP, PE, PS, PC und/oder PMMA handeln.

Das erste Bauteil weist zwei miteinander verbundene Elemente auf, wobei insbesondere ein erstes Element den thermoplastischen Kunststoff und ein zweites Element das Elastomer umfasst.

Das erste Element kann dabei im Spritzgussverfahren hergestellt sein. Mit anderen Worten kann das Verfahren ein Herstellen eines ersten Elements aus einem thermoplastischen Kunststoff mittels Spritzgießen umfassen.

Das erste Element kann insbesondere aus dem thermoplastischen Kunststoff bestehen.

Das erste Element kann jedoch auch andere Materialien umfassen oder aus anderen Materialien bestehen, beispielsweise Glas. Mit anderen Worten weist das erste Bauteil zwei miteinander verbundene Elemente auf, wobei eines der Elemente das Elastomer umfasst.

Das zweite Element umfasst das Elastomer. Das zweite Element kann aus dem Elastomer bestehen. Das zweite Element kann dabei in einem Zweikomponentenspritzgussverfahren an das erste Element angespritzt werden.

Das erste Bauteil kann insbesondere eine rechteckige oder kreisförmige Grundfläche aufweisen. Das erste Element und das zweite Element können dann ebenfalls rechteckig oder kreisförmig ausgebildet sein, insbesondere wobei das zweite Element an der Unterseite oder Oberseite des ersten Elements angeordnet ist.

Das zweite Bauteil kann entsprechend ebenfalls rechteckig oder kreisförmig ausgebildet sein.

Die maximale oder mittlere Ausdehnung des zweiten Elements in einer Richtung senkrecht zur Kontaktfläche des ersten Bauteils kann maximal 50%, insbesondere von 1% bis 20%, der gesamten maximalen oder mittleren Ausdehnung des ersten Bauteils senkrecht zur Kontaktfläche des ersten Bauteils betragen. In einer nicht unter die Erfindung fallenden Alternative, in der das zweite Element nicht in Richtung des Reservoirs abgedeckt wird, kann dadurch der Bereich minimiert werden, in dem eine Probe in der Probenkammer, insbesondere in Form einer Flüssigkeit, mit dem Elastomer in Kontakt kommt. Das Elastomer kann dazu neigen, Flüssigkeiten aufzunehmen oder Komponenten enthalten, die in die Flüssigkeit diffundieren können. Durch die Minimierung der Ausdehnung des zweiten Elements kann das Kontaminierungsrisiko verhindert werden.

Das erste Element kann insbesondere eine maximale oder mittlere Ausdehnung senkrecht zur Kontaktfläche des ersten Bauteils von 1 cm oder mehr als 1 cm aufweisen. Das zweite Element kann eine maximale oder mittlere Ausdehnung senkrecht zur Kontaktfläche des ersten Bauteils von weniger als 1 cm, insbesondere weniger als 1 mm, aufweisen.

Das zweite Element und/oder das erste Bauteil können auch eine konstante Ausdehnung haben. In diesem Fall ist die maximale Ausdehnung gleich der mittleren Ausdehnung gleich der konstanten Ausdehnung.

Eine typische Ausdehnung des ersten Elementes senkrecht zur Kontaktfläche des ersten Bauteils kann beispielsweise 2 cm, die des zweiten Elementes 1 mm betragen. Wenn das zweite Element als Septum genutzt werden soll, kann die Ausdehnung des zweiten Elements senkrecht zur Kontaktfläche des ersten Bauteils zumindest teilweise auch größer sein, insbesondere in dem Bereich des zweiten Elements, das als Septum genutzt werden soll. Insbesondere kann die Ausdehnung des zweiten Elements senkrecht zur Kontaktfläche des ersten Bauteils auch variieren.

In einer besonderen Ausführung, die nicht unter die Erfindung fällt, können auch Elastomerelemente, die nur als Septum dienen sollen, in den Thermoplastbereich des ersten Bauteils integriert sein. Mit anderen Worten kann das erste Element Bereiche umfassen, die ein Elastomer umfassen oder aus Elastomer bestehen, und an ein Reservoir der Probenkammer angrenzen, das durch das Verbinden des ersten und des zweiten Bauteils gebildet wird. Diese Bereiche können teilweise oder vollständig von Bereichen des ersten Elements umgeben sein, die kein Elastomer umfassen. Mit anderen Worten müssen diese Bereiche dann keinen Kontakt zum zweiten Bauteil haben. Diese Septen können eine Ausdehnung von 2 cm bis 5 mm haben.

Diese Bereiche können insbesondere unmittelbar an das Reservoir angrenzen. Die Bereiche können insbesondere durchgehend sein und die Außenseite des Reservoirs mit der Innenseite verbinden.

Solche Bereiche sind auch für Probenkammern denkbar, die nicht durch das hier beschriebene Verfahren hergestellt wurden. Beispielsweise können diese Bereiche auch in Probenkammern vorgesehen sein, bei denen die Bauteile miteinander verklebt oder verschweißt sind, bzw. bei einkomponentigen Probenkammern.

Das erste Bauteil bildet nach dem Verbinden eine Seitenwand und das zweite Bauteil einen Boden eines Reservoirs. In einer nicht unter die Erfindung fallenden Alternative kann das erste Bauteil nach dem Verbinden des ersten Bauteils mit dem zweiten Bauteil einen Boden und das zweite Bauteil eine Seitenwand eines Reservoirs bilden.

Als Reservoir kann hierin insbesondere ein Volumensbereich der herzustellenden Probenkammer verstanden werden, der zum Aufnehmen einer Flüssigkeit geeignet ist. Dazu kann das Reservoir wenigstens eine Seitenwand und einen Boden umfassen. Dabei kann es sich um ein nach oben offenes Reservoir oder um einen Hohlraum handeln.

Durch das Verbinden des ersten und des zweiten Bauteils wird ein Reservoir erhalten , wobei das erste Bauteil eine Seitenwand des Reservoirs bildet, wobei das erste Bauteil ein erstes und ein damit verbundenes zweites Element aufweist, wobei nur das zweite Element das Elastomer umfasst, und wobei das erste Element so angeordnet ist, dass es das zweite Element zum Reservoir hin wenigstens teilweise verdeckt oder abdeckt. Dadurch kann das Kontaminierungsrisiko im Reservoir weiter verringert werden. In diesem Fall kann das erste Element insbesondere einen thermoplastischen Kunststoff umfassen oder aus einem thermoplastischen Kunststoff bestehen. Das erste Element kann das zweite Element insbesondere so abdecken, dass es das zweite Element unmittelbar berührt. Das erste Element kann auch so angeordnet sein, dass es das zweite Element zum Reservoir hin vollständig abdeckt.

Zusätzlich kann durch das Verbinden des ersten und des zweiten Bauteils ein Reservoir erhalten werden, wobei das erste Bauteil eine Seitenwand des Reservoirs bildet, wobei das erste Bauteil ein erstes und ein damit verbundenes zweites Element aufweist, wobei nur das zweite Element das Elastomer umfasst, und wobei das erste Element so angeordnet ist, dass es das zweite Element auf einer dem Reservoir abgewandten Seite wenigstens teilweise verdeckt oder abdeckt. Dadurch können mögliche Beschädigungen des Elastomers verhindert oder verringert werden. In diesem Fall kann das erste Element insbesondere einen thermoplastischen Kunststoff umfassen oder aus einem thermoplastischen Kunststoff bestehen. Das erste Element kann das zweite Element insbesondere so abdecken, dass es das zweite Element unmittelbar berührt. Das erste Element kann auch so angeordnet sein, dass es das zweite Element auf der vom Reservoir abgewandten Seite vollständig abdeckt.

Das erste Element kann auch so angeordnet sein, dass es auch eine Seitenwand des zweiten Bauteils wenigstens teilweise verdeckt oder abdeckt, insbesondere unmittelbar. Dadurch kann auch das zweite Bauteil wenigstens teilweise vor Beschädigungen geschützt werden.

Alternativ oder zusätzlich zum Behandeln des Elastomers im Bereich einer Kontaktfläche des ersten Bauteils kann das Verfahren ein Behandeln der Kontaktfläche des zweiten Bauteils mit einem Plasma, mit Plasmaabgas und/oder mit einem reaktiven Gas umfassen. Es hat sich gezeigt, dass auch nur eine Behandlung der Kontaktfläche des zweiten Bauteils für eine sichere Verbindung ausreichend ist. Es können aber auch beide Kontaktflächen behandelt werden. Dadurch kann die Verbindung zwischen dem ersten und dem zweiten Bauteil weiter verstärkt werden.

Das Verfahren kann ein Bestrahlen der Kontaktfläche des zweiten Bauteils mit UV-Licht umfassen. Dadurch ist eine weitere Verbesserung der Verbindung der Bauteile möglich. Es kann auch gleichzeitig oder hintereinander ein Bestrahlen mit UV-Licht und ein Behandeln mit Plasma/Plasmaabgasen/reaktivem Gas erfolgen.

Das Verfahren kann ein Reinigen des zweiten Bauteils vor dem Verbinden, insbesondere in einem Ultraschallbad mit Wasser, Isopropanol, einer Säure und/oder einer Lauge umfassen. Auch dadurch ist es möglich, eine bessere Verbindung zwischen den Bauteilen zu erreichen.

Das Verbinden der Kontaktfläche des ersten Bauteils mit einer Kontaktfläche des zweiten Bauteils kann insbesondere ein Anpressen der Kontaktfläche des ersten Bauteils an die Kontaktfläche des zweiten Bauteils umfassen, insbesondere mit einer Kraft von wenig als 20 N, insbesondere weniger als 5 N. Dadurch kann ohne den Aufwand von großen mechanischen Kräften eine sichere Verbindung zwischen den Bauteilen hergestellt werden. Insbesondere durch die geringen aufzuwendenden mechanischen Kräfte kann die Herstellung der Probekammer erleichtert werden. Dabei haben die Erfinder der vorliegenden Anmeldung überraschend festgestellt, dass eine Kraft von weniger als 20 N, insbesondere weniger als 5 N, ausreichen kann, um die sichere Verbindung herzustellen.

Das erste Bauteil, das nach dem Verbinden eine Seitenwand eines Reservoirs bildet, kann eine Kante, einen Vorsprung und/oder eine Einbuchtung zum Auflegen eines Deckels umfassen. Dadurch kann ein sichererer Sitz eines Deckels gewährleistet werden. Die Kante, der Vorsprung und/oder die Einbuchtung kann insbesondere umlaufend ausgebildet sein.

Das Verfahren kann außerdem ein Bereitstellen eines dritten Bauteils und Verbinden des dritten Bauteils mit dem ersten Bauteil umfassen, insbesondere wobei das Elastomer im Bereich einer weiteren Kontaktfläche des ersten Bauteils vor dem Verbinden mit einem Plasma, mit Plasmaabgas und/oder mit einem reaktiven Gas behandelt wird. Mit anderen Worten kann das dritte Bauteil auf dieselbe Weise wie das zweite Bauteil mit dem ersten Bauteil verbunden werden.

Das dritte Bauteil kann eines oder mehrere der oben genannten Merkmale des zweiten Bauteils umfassen. Insbesondere kann eine Kontaktfläche des dritten Bauteils vor dem Verbinden, wie oben für die Kontaktfläche des zweiten Bauteils beschrieben, behandelt werden. Das Verbinden des dritten Bauteils mit dem ersten Bauteil kann eines oder mehrere der oben genannten Merkmale für das Verbinden des ersten Bauteils mit dem zweiten Bauteil umfassen.

Das dritte Bauteil kann nach dem Verbinden beispielsweise eine Deckplatte der Probenkammer bilden.

Das erste Bauteil kann so ausgebildet sein, dass ein Teil des ersten Bauteils, in dem ein Teil des Elastomers angeordnet ist, nach dem Verbinden des ersten mit dem zweiten Bauteil über eine Seitenfläche des zweiten Bauteils hinausragt. Dadurch kann das zweite Bauteil vor Beschädigungen geschützt werden. Dies ist insbesondere von Vorteil, wenn das zweite Bauteil ein Glas umfasst und aus Glas besteht, und die hergestellten Probenkammern nach der Herstellung als Schüttgut gelagert werden sollen.

Dieser Teil des ersten Bauteils kann insbesondere so ausgebildet sein, dass er sich nach dem Verbinden in eine Richtung senkrecht zur Kontaktfläche des ersten Bauteils erstreckt, insbesondere so, dass eine Seitenfläche des zweiten Bauteils wenigstens teilweise abgedeckt oder überlappt wird. Dadurch kann ein noch besserer Schutz gewährleistet werden. Die Seitenfläche des zweiten Bauteils kann dabei mit dem Teil des ersten Bauteils in Kontakt stehen, insbesondere in direktem Kontakt. Alternativ kann der Teil des ersten Bauteils die Seitenfläche des zweiten Bauteils beabstandet wenigstens teilweise abdecken oder überlappen.

Insbesondere kann der Teil so ausgebildet sein, dass er nach dem Verbinden über eine der Kontaktfläche des zweiten Bauteils abgewandte Seite des zweiten Bauteils hinausragt. Dadurch kann ein noch besserer Schutz des zweiten Bauteils erreicht werden.

Das erste Bauteil kann nach dem Verbinden eine Seitenwand und das zweite Bauteil einen Boden eines Reservoirs bilden, wobei das erste Bauteil über eine Seitenfläche des zweiten Bauteils hinausragt und eine Stufe oder Erhöhung aufweist, die sich in einer Richtung senkrecht zur Kontaktfläche des ersten Bauteils erstreckt, und wobei die Stufe oder Erhöhung eine Seitenfläche des zweiten Bauteils wenigstens teilweise, insbesondere beabstandet, abdeckt oder überlappt. Die Stufe oder Erhöhung kann auch eine Ausdehnung senkrecht zur Kontaktfläche des ersten Bauteils aufweisen, die größer ist als die maximale oder mittlere oder konstante Dicke des zweiten Bauteils. Dadurch kann die Stufe oder Erhöhung über die Unterseite des zweiten Bauteils hinausragen.

Bei dem zweiten Bauteil kann es sich insbesondere um ein Deckglas mit einer (konstanten) Dicke von 1 µm bis 300 µm, insbesondere 100 µm bis 200 µm, handeln. Eine Probenkammer mit einem solchen zweiten Bauteil, insbesondere als Bodenplatte, ermöglicht in vorteilhafter Weise eine Anwendung der inversen Mikroskopie, insbesondere der hochauflösenden Mikroskopie.

Die Kontaktfläche des ersten Bauteils kann glatt, insbesondere ohne Strukturelemente, ausgebildet sein. Insbesondere kann die Oberfläche so glatt wie ein Float Glas ausgebildet sein. Dadurch kann die Verbindung zwischen den Bauteilen verbessert werden.

Die Rauheit der Kontaktfläche des ersten und/oder des zweiten Bauteils kann insbesondere kleiner als 1 nm, insbesondere kleiner als 0.5 nm, sein. Die Rauheit kann dabei gemäß EN ISO 25178 bestimmt werden.

Das erste Bauteil kann unterschiedliche Dicken aufweisen. Insbesondere kann die Dicke des ersten Bauteils in einem Bereich, der wenigstens einen Teil des Elastomers umfasst oder aus wenigstens einem Teil des Elastomers besteht, geringer sein als die mittlere Dicke des ersten Bauteils. Dieser Bereich des ersten Bauteils kann insbesondere nach dem Verbinden des ersten Bauteils mit dem zweiten Bauteil an ein Reservoir angrenzen. Dieser Bereich kann dann als Zugang für eine Spritze, eine Kanüle oder zum Einbringen von Lueranschlüssen geeignet sein. Insbesondere kann dieser Bereich als Septum oder Durchstichmembran dienen.

Dieser Bereich kann insbesondere unmittelbar an das Reservoir angrenzen. Der Bereich kann insbesondere durchgehend sein und die Außenseite des Reservoirs mit der Innenseite verbinden. Es können auch mehrere solche Bereiche vorgesehen sein.

In dem Bereich kann das erste Bauteil insbesondere eine Dicke von weniger als 2 mm, insbesondere weniger als 1 mm, aufweisen.

Beispielsweise können Proben dann durch eine Nadel in das Reservoir eingebracht werden, wobei der Bereich des ersten Bauteils gleichzeitig die Funktion eines Septums einnimmt.

Insbesondere kann das erste Bauteil ein erstes und ein damit verbundenes zweites Element aufweist, wobei das zweite Element das Elastomer umfasst oder aus dem Elastomer besteht, und wobei das zweite Element in einem oder mehreren Bereichen, die nach dem Verbinden der Bauteile an ein Reservoir, insbesondere unmittelbar, angrenzen, eine geringere Dicke aufweist als das erste Element. Das erste Element kann eines oder mehrere der oben genannten Merkmale aufweisen.

Das Verfahren kann insbesondere ein Verfahren zum Herstellen einer Probenkammer mit einem oder mehreren Reservoirs sein. Insbesondere können 1, 12, 24, 48, 96, 384 oder 1536 runde oder rechteckige Reservoirs gebildet werden. Diese können, je nach Ausführung, den gängigen Standards entsprechen.

Das Bauteil, das nach dem Verbinden den Boden eines Reservoirs bildet, kann auch im Bereich des Bodens des Reservoirs beschichtet oder chemisch oder physikalisch behandelt sein, so dass optimale Eigenschaften für das Zellwachstum generiert werden. Dabei können insbesondere NH, COOH oder andere hydrophile Gruppen verwendet werden. Es kann sich aber auch um plasmabehandelten Kunststoff handeln. Mit anderen Worten kann das Bauteil, das nach dem Verbinden den Boden eines Reservoirs bildet, im Bereich des Bodens des Reservoirs plasmabehandelt werden, bevor die Bauteile verbunden werden.

Die Probenkammer kann auch im Spritzgussverfahren mit direkter Aufspritzung auf eine Bodenplatte erfolgen. Dazu wird eine Bodenplatte, z.B. ein Deckglas der Stärke 1, 5, in ein Spritzgusswerkzeug eingelegt. Dabei kann sowohl eine Thermoplast direkt auf die Bodenplatte aufgespritzt werden, wobei die Bodenplatte insbesondere, wie zuvor oben beschrieben, behandelt oder aktiviert wurde. Es können aber auch zwei Komponentenspritzgussverfahren in Kombination mit einer eingelegten Bodenplatte verwendet werden.

Die Erfindung stellt außerdem eine Probenkammer für Mikroskopuntersuchungen nach Anspruch 12 bereit, erhalten durch ein oben beschriebenes Verfahren.

Bei einer derartigen Probenkammer kann auf Klebstoff oder eine adhäsive Schicht verzichtet werden. Dadurch kann eine Kontamination der Probe mit Lösungsmittelrückständen verhindert werden.

Die Probenkammer, insbesondere das erste und das zweite Bauteil, können eines oder mehrere der oben genannten Merkmale aufweisen.

Weitere Merkmale und Vorteile der Erfindung werden nachfolgend anhand der beispielhaften Figuren erläutert. Dabei zeigt:
- Figur 1: eine perspektivische Ansicht einer beispielhaften Probenkammer;
- Figuren 2a und 2b: Querschnittsansichten der beispielhaften Probenkammer aus Figur 1;
- Figur 3: eine Querschnittsansicht einer weiteren beispielhaften Probenkammer;
- Figur 4: eine perspektivische Ansicht einer weiteren beispielhaften Probenkammer;
- Figuren 5a und 5b: Querschnittsansichten der beispielhaften Probenkammer aus Figur 4;
- Figuren 6a und 6b: Querschnittsansichten einer weiteren beispielhaften Probenkammer;
- Figuren 7a und 7b: perspektivische Ansichten einer weiteren beispielhaften Probenkammer;
- Figuren 8a und 8b: Querschnittsansichten einer erfindungsgemäßen Probenkammer (Figur 8a) und einer weiteren beispielhaften Probenkammer (Figur 8b); und
- Figur 9: eine Querschnittsansicht einer weiteren beispielhaften Probenkammer.

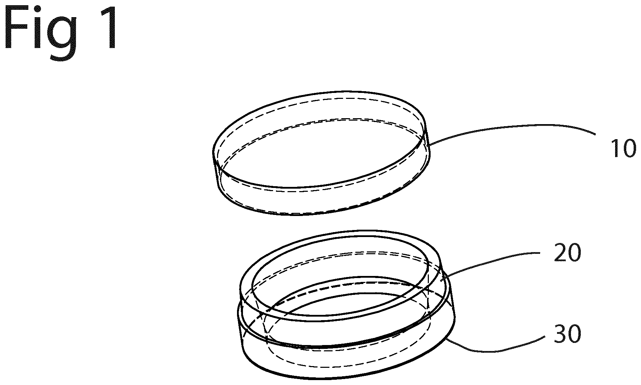

In Figur 1 ist eine beispielhafte Probenkammer in perspektivischer Darstellung gezeigt. Diese Probenkammer kann beispielsweise für Mikroskopuntersuchungen, beispielsweise für Fluoreszenzmikroskopie, verwendet werden. Die beispielhafte Probenkammer umfasst ein erstes Bauteil 20 und ein zweites Bauteil 30. Das erste Bauteil 20 besteht in diesem Beispiel aus einem Elastomer, beispielsweise aus einem Silikon, insbesondere aus Polydimethylsiloxan (PDMS).

Das Elastomer kann auch ein auf Elastomer basiertes Spritzguss-Silikon sein, wie es beispielsweise durch die Firma Wacker Chemie AG unter der Bezeichnung "Elastosil" vertrieben wird.

Das zweite Bauteil 30 besteht in diesem Beispiel aus Glas, beispielsweise Borosilikatglas, Quarzglas oder Kalk-Natron Glas.

Alternativ könnte das zweite Bauteil 30 auch eine Kunststofffolie sein, also ein Kunststoffelement, dessen Längsausdehnungen deutlich größer sind als dessen Dicke. Diese Kunststofffolie könnte insbesondere aus COC, COP, PE, PS, PC und/oder PMMA bestehen. Diese Kunststofffolie könnte dabei vollflächig oder teilweise mit einem Glas bedampft sein, insbesondere mit SIOx.

Das erste Bauteil 20 weist in diesem Beispiel eine Kontaktfläche auf, in der das erste Bauteil 20 mit dem zweiten Bauteil 30 verbunden ist. Das zweite Bauteil 30 weist eine entsprechende Kontaktfläche auf.

Die Kontaktfläche des ersten Bauteils 20 ist dabei vor dem Verbinden mit dem zweiten Bauteil 30 mit einem Plasma, mit Plasmaabgas und/oder mit einem reaktiven Gas behandelt worden. Wenn das zweite Bauteil 30 eine glasbedampfte Kunststofffolie ist, ist das Glas wenigstens im Bereich der Kontaktfläche des zweiten Bauteils 30 angeordnet. Das Glas kann auch nur im Bereich der Kontaktfläche des zweiten Bauteils 30 angeordnet sein.

Dadurch, dass das Elastomer im Bereich der Kontaktfläche des ersten Bauteils 20 mit einem Plasma, mit Plasmaabgas und/oder mit einem reaktiven Gas behandelt wurde ist zwischen dem ersten Bauteil 20 und dem zweiten Bauteil 30 eine dauerhafte, flüssigkeitsdichte Verbindung geschaffen, die ohne Klebstoff oder adhäsiver Schicht gebildet wurde. Somit konnte die Verbindung zwischen den Bauteilen lösungsmittelfrei hergestellt werden. Auch ein energieaufwendiges Ultraschallverschweißen ist nicht erforderlich.

Mit anderen Worten kann die Probenkammer aus Figur 1 durch folgendes Verfahren hergestellt werden:
Zunächst wird das erste Bauteil 20 und das zweite Bauteil 30 bereitgestellt, wobei das erste Bauteil 20 aus einem Elastomer besteht.

Das Elastomer wird dann im Bereich einer Kontaktfläche des ersten Bauteils 20 mit einem Plasma, einer Korona, einem Atmosphärenplasma oder mit Plasmaabgas und/oder mit einem reaktiven Gas behandelt.

Die derart behandelte Kontaktfläche des ersten Bauteils 20 wird dann mit einer entsprechenden Kontaktfläche des zweiten Bauteils 30 verbunden. Das Verbinden kann ein Anpressen der Kontaktsfläche des ersten Bauteils 20 an die Kontaktfläche des zweiten Bauteils 30 umfassen, insbesondere mit einer geringen Kraft von weniger als 20 N, insbesondere weniger als 5 N.

Alternativ oder zusätzlich könnte auch die Kontaktfläche des zweiten Bauteils 30 mit einem Plasma, einer Korona, einem Atmosphärenplasma oder mit Plasmaabgas und/oder mit einem reaktiven Gas behandelt werden.

In Figur 1 ist außerdem ein Deckel 10 zum Verschließen eines Reservoirs der Probenkammer gezeigt. Durch einen solchen Deckel 10 kann das Verdunsten von Flüssigkeit aus dem Reservoir vermieden oder wenigstens reduziert werden. Außerdem kann durch den Deckel eine Kontaminierung der Probe von Außen verhindert oder wenigstens reduziert werden. Damit kann eine innen sterile Zellkulturkammer, Petrischale oder Multiwellplatte bereitgestellt werden.

Das erste Bauteil 20 weist eine umlaufende Kante 60 zum Auflegen des Deckels 10 auf. Die umlaufende Kante 60 ist dabei derart ausgebildet, dass der Deckel wenigsten teilweise an der seitlichen Außenfläche des ersten Bauteils 20 flächig anliegt. Die Kante 60 kann insbesondere eine Breite aufweisen, die der Dicke des Materials des Deckels 10 im Bereich der Auflage auf der Kante 60 entspricht. Dadurch kann erreicht werden, dass der Deckel 10 mit der Außenwand des ersten Bauteils 20 nach dem Aufsetzen fluchtet. Dadurch kann eine einfachere Handhabung der Probenkammer ermöglicht werden. Auch eine nicht fluchtende Ausbildung ist jedoch denkbar.

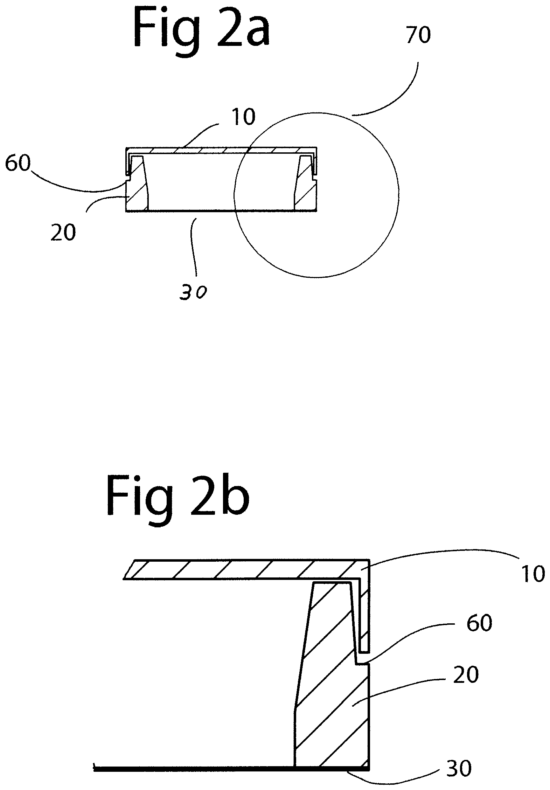

Figuren 2a und 2b zeigen Querschnittsansichten der beispielhaften Probenkammer aus Figur 1. Figur 2a zeigt insbesondere einen kompletten Querschnitt der Probenkammer während in Figur 2b eine vergrößerte Ansicht des Ausschnitts 70 aus Figur 2a dargestellt ist.

In Figur 2a sind nochmals das erste Bauteil 20, das zweite Bauteil 30 und der Deckel 10 gezeigt. Der Deckel 10 ist in Figur 2a auf das erste Bauteil 20 aufgesetzt. Dabei ist die umlaufende Kante 60 gezeigt, die ein Auflegen des Deckels 10 ermöglicht.

In Figur 2b ist der Bereich 70 vergrößert dargestellt. Die Oberfläche des ersten Bauteils 20, die in diesem verbundenen Zustand das zweite Bauteil 30 berührt oder kontaktiert, wird als Kontaktfläche des ersten Bauteils 20 bezeichnet. Diese Kontaktfläche wurde vor dem Verbinden der Bauteile mit einem Plasma, mit Plasmaabgas und/oder mit einem reaktiven Gas behandelt. Der Oberflächenbereich des zweiten Bauteils 30, der durch das erste Bauteil 20 abgedeckt oder berührt wird, wird als Kontaktfläche des zweiten Bauteils 30 bezeichnet. Diese Kontaktfläche kann ebenfalls vor dem Verbinden mit einem Plasma, mit Plasmaabgas und/oder einem reaktiven Gas behandelt worden sein.

Alternativ oder zusätzlich kann die Kontaktfläche des zweiten Bauteils 30 vor dem Verbinden mit einem UV-Licht bestrahlt worden sein. In beiden Fällen kann eine noch bessere Verbindung zwischen den Bauteilen erreicht werden.

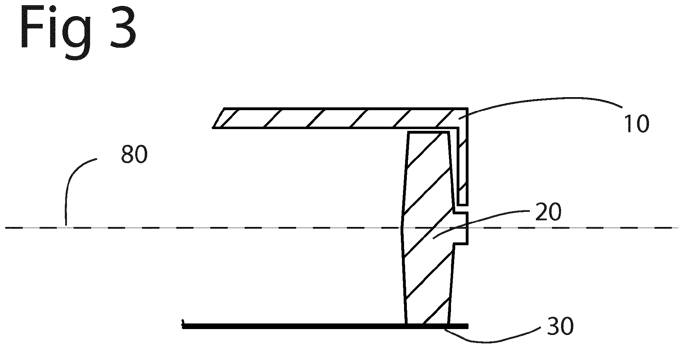

Figur 3 zeigt einen Ausschnitt aus einer Querschnittsansicht für eine weitere beispielhafte Probenkammer. Auch diese Probenkammer umfasst ein erstes Bauteil 20, das aus einem Elastomer besteht, und ein zweites Bauteil 30. Das zweite Bauteil 30 kann wie oben beschrieben ausgebildet sein. Insbesondere kann es sich bei dem zweiten Bauteil 30 um ein Deckglas mit einer Dicke von 1 µm bis 500 µm, insbesondere von 100 µm bis 200 µm, handeln.

Wie auch in den vorherigen Beispielen wurde eine Kontaktfläche des ersten Bauteils 20 vor dem Verbinden der Bauteile mit einem Plasma, mit Plasmaabgas und/oder mit einem reaktiven Gas behandelt.

In diesem Beispiel ist das erste Bauteil 20 bezüglich einer zur Kontaktfläche des ersten Bauteils 20 parallelen Symmetrieebene 80 spiegelsymmetrisch ausgebildet. Dies erleichtert die Produktion, da während des Produktionsprozesses das zweite Bauteil 30 nicht auf eine bestimmte Seite des ersten Bauteils 20 aufgebracht werden muss, sondern zwei gleichwertige Flächen zur Verbindung bereitstehen.

In der Figur 3 ist die Symmetrieebene 80 zur Illustration als gestrichelte Linie angedeutet.

Die Kontaktfläche des zweiten Bauteils 30 ist in diesem Beispiel seitlich von weiteren Oberflächenbereichen des zweiten Bauteils 30 umgeben. Mit anderen Worten liegt die Kontaktfläche des zweiten Bauteils 30 nicht an einem Rand des zweiten Bauteils 30. Außerdem ist das zweite Bauteil in diesem Beispiel so ausgebildet, dass ein Teil des zweiten Bauteils 30 in Figur 3 über die äußere Seitenfläche des ersten Bauteils 20 hinaus.

Alternativ kann auch das erste Bauteil 20 wenigstens teilweise über eine Seitenfläche des zweiten Bauteils 30 hinausragen.

Gemäß einer Alternative könnte die Kontaktfläche des zweiten Bauteils 30 auch an einem Rand des zweiten Bauteils 30 angeordnet sein. In diesem Fall kann die Seitenfläche des ersten Bauteils 20 nach dem Verbinden insbesondere mit einer Seitenfläche des zweiten Bauteils 30 fluchten.

In den bisherigen Beispielen bestand das erste Bauteil 20 aus einem Elastomer. Alternativ könnte das erste Bauteil 20 jedoch neben dem Elastomer auch noch weitere Materialien umfassen. Insbesondere kann das erste Bauteil 20 zusätzlich einen thermoplastischen Kunststoff, insbesondere COC, COP, PE, PS, PC und/oder PMMA umfassen. In diesem Fall ist das erste Bauteil 20 jedoch so ausgebildet, dass das Elastomer wenigstens im Bereich der Kontaktfläche des ersten Bauteils 20, die mit einem Plasma, mit Plasmaabgas und/oder mit einem reaktiven Gas behandelt wird, angeordnet ist.

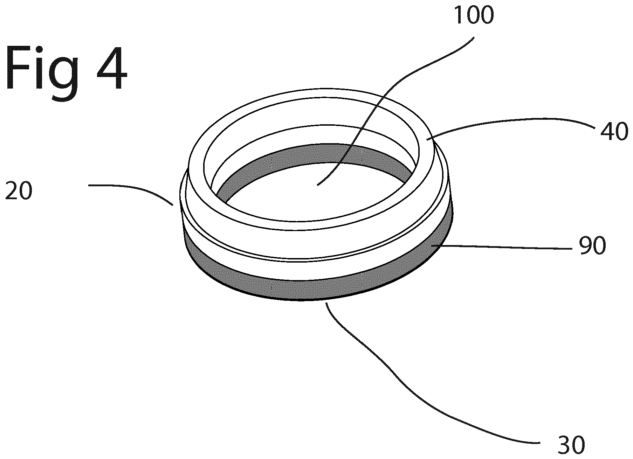

Das erste Bauteil 20 kann auch mehrkomponentig ausgebildet sein. Ein entsprechendes Beispiel einer Probenkammer ist in Figur 4 dargestellt.

In diesem Beispiel umfasst das erste Bauteil 20 ein erstes Element 40 und ein zweites Element 90. Das zweite Element 90 umfasst das Elastomer oder besteht aus einem Elastomer, während das erste Element 40 einen thermoplastischen Kunststoff umfasst oder aus einem thermoplastischen Kunststoff besteht. In diesem Beispiel kann das erste Bauteil 20 in einem Zweikomponentenspritzgußverfahren hergestellt worden sein.

Die Verbindung mit dem zweiten Bauteil 30 erfolgte dann, wie in den obigen Beispielen beschrieben.

Durch die Kombination eines thermoplastischen Kunststoffs und eines Elastomers kann das erste Bauteil 20 stabiler ausgebildet sein.

Die, in diesem Beispiel konstante, Ausdehnung des zweiten Elements 90 in einer Richtung senkrecht zur Kontaktfläche des ersten Bauteils kann maximal 50 %, insbesondere von 1 % bis 20 %, der gesamten, in diesem Beispiel konstanten, Ausdehnung des ersten Bauteils 20 senkrecht zur Kontaktfläche betragen.

Mit anderen Worten kann die Höhe des zweiten Elements 90 maximal die Hälfte, insbesondere nur 1 % bis 20 %, der Gesamthöhe des ersten Bauteils 20 betragen.

Durch eine derartige Ausgestaltung des zweiten Elements 90 kann der Kontakt zwischen einer Flüssigkeit, die in einem Reservoir 100 angeordnet wird, das sich durch das Verbinden des ersten Bauteils 20 mit dem zweiten Bauteil 30 bildet, und dem Elastomer minimiert werden. Dies ist vorteilhaft, da das Elastomer dazu neigen kann, Flüssigkeiten aufzunehmen oder Komponenten in eine Flüssigkeit abzugeben. Durch die niedrige Höhe des zweiten Elements 90 kann das Kontaminierungsrisiko in der Flüssigkeit also minimiert werden.

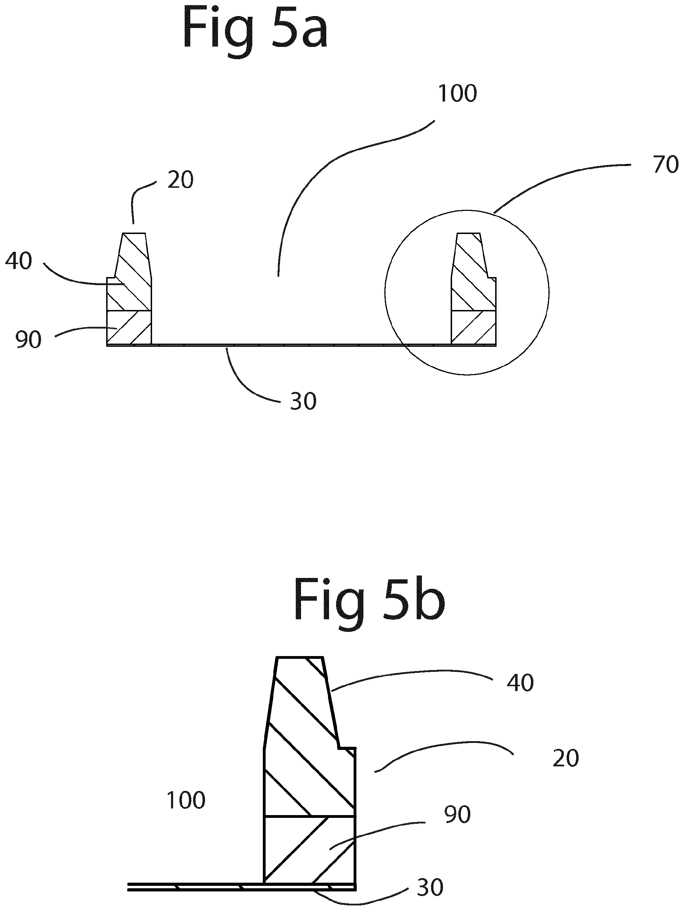

In den Figuren 5a und 5b ist die beispielhafte Probenkammer aus Figur 4 nochmals in Querschnittsansichten gezeigt. Insbesondere zeigt Figur 5b eine vergrößerte Ansicht des Bereichs 70 aus Figur 5a.

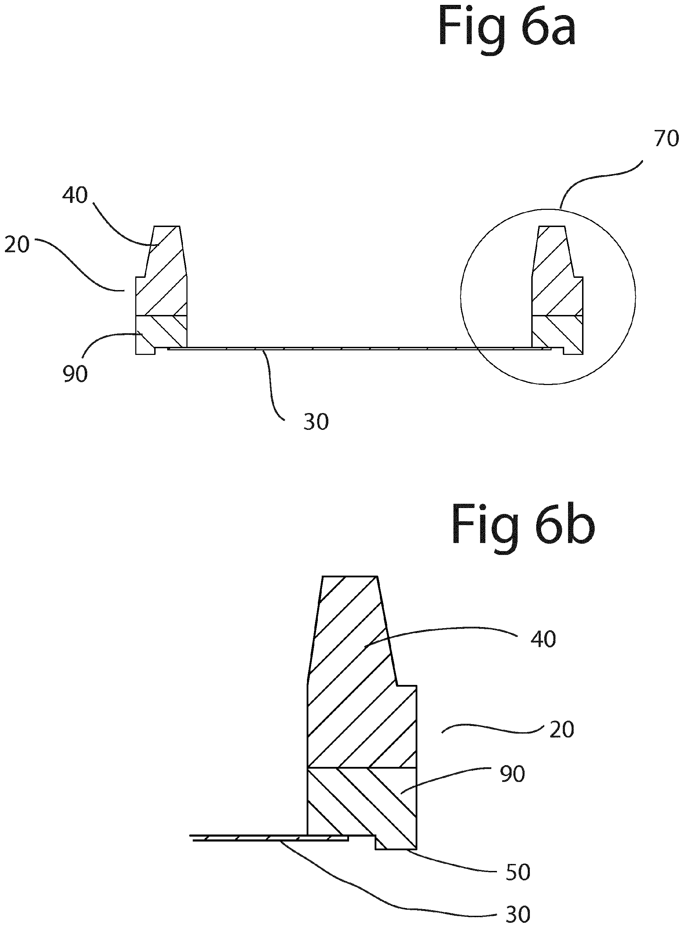

In Figuren 6a und 6b sind Querschnittsansichten einer weiteren beispielhaften Probenkammer gezeigt. Insbesondere ist in Figur 6b eine vergrößerte Darstellung des Bereichs 70 aus Figur 6a dargestellt. Auch diese Probenkammer umfasst ein erstes Bauteil 20 und ein zweites Bauteil 30, die wie oben dargelegt verbunden wurden. Das erste Bauteil 20 umfasst dabei ein erstes Element 40 und ein zweites Element 90, wobei das zweite Element 90 das Elastomer umfasst oder aus dem Elastomer besteht.

Wie insbesondere in der Detailansicht in Figur 6b zu sehen ist, ist das erste Bauteil 20, insbesondere das zweite Element 90, so ausgebildet, dass ein Teil 50 des ersten Bauteils 20 über eine Seitenfläche des zweiten Bauteils 30 hinausragt.

In diesem Beispiel ragt der Teil 50 auch über eine der Kontaktfläche des zweiten Bauteils 30 abgewandte Seite des zweiten Bauteils 30 hinaus. Dadurch kann das zweite Bauteil 30 vor Beschädigungen geschützt werden. Dies ist insbesondere von Vorteil, wenn die Probenkammern nach deren Herstellung als Schüttgut gelagert werden. Besonders vorteilhaft ist dies, wenn das zweite Bauteil 30 Glas umfasst oder aus Glas besteht, da das Elastomer nicht in der Lage ist, dass Glas zu brechen. Durch den Teil 50 können Glas-Glas Kontakte im Schüttgut vermieden oder vermindert werden.

In den bisherigen Beispielen umfasste die Probenkammer ein Reservoir 100 zum Aufnehmen einer Flüssigkeit. Es können jedoch auch zwei oder mehr Reservoire vorgesehen sein, welche insbesondere durch das Verbinden des ersten Bauteils 20 mit dem zweiten Bauteil 30 gebildet werden. Insbesondere können 12, 24, 48, 96, 384 oder 1536 Reservoire vorgesehen sein.

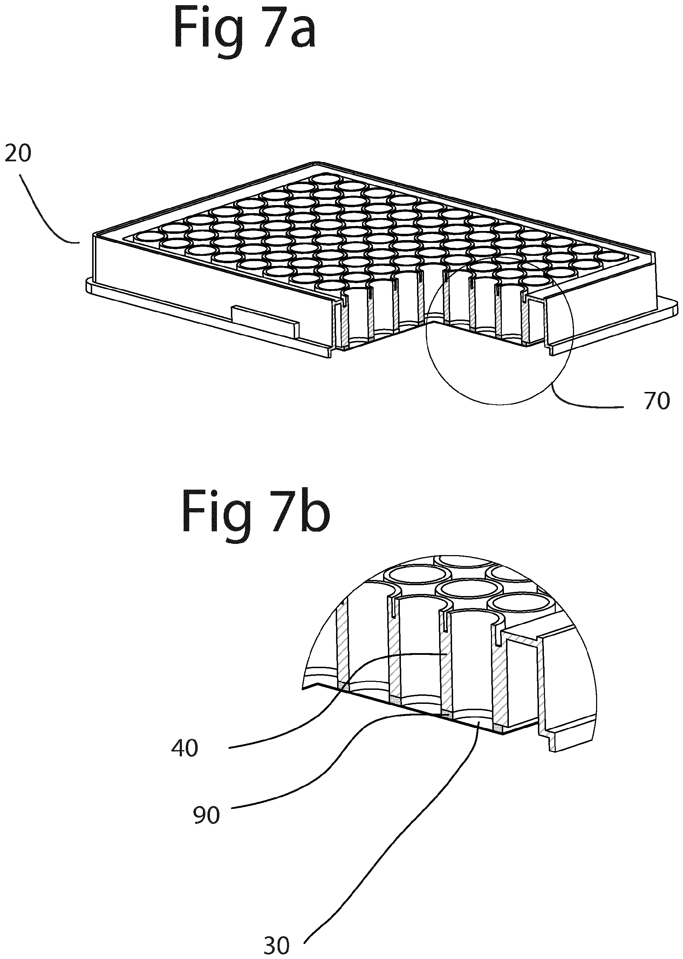

Ein entsprechendes Beispiel für eine Probenkammer mit mehreren Reservoiren ist in den Figuren 7a und 7b dargestellt. In Figur 7b ist insbesondere eine Detailansicht des Bereiches 70 aus Figur 7a gezeigt.

Auch diese beispielhafte Probenkammer umfasst ein erstes Bauteil 20 und ein zweites Bauteil 30, die wie oben beschrieben verbunden wurden. Insbesondere umfasst das erste Bauteil 20 ein erstes Element 40, umfassend einen thermoplastischen Kunststoff, und ein zweites Element 90, das ein Elastomer umfasst oder aus Elastomer besteht. Im ersten Bauteil 20 sind eine Vielzahl von Durchgangsöffnungen ausgebildet, die nach dem Verbinden des ersten Bauteils 20 mit dem zweiten Bauteil 30 eine Vielzahl von Reservoiren zum Aufnehmen einer Flüssigkeit bilden.

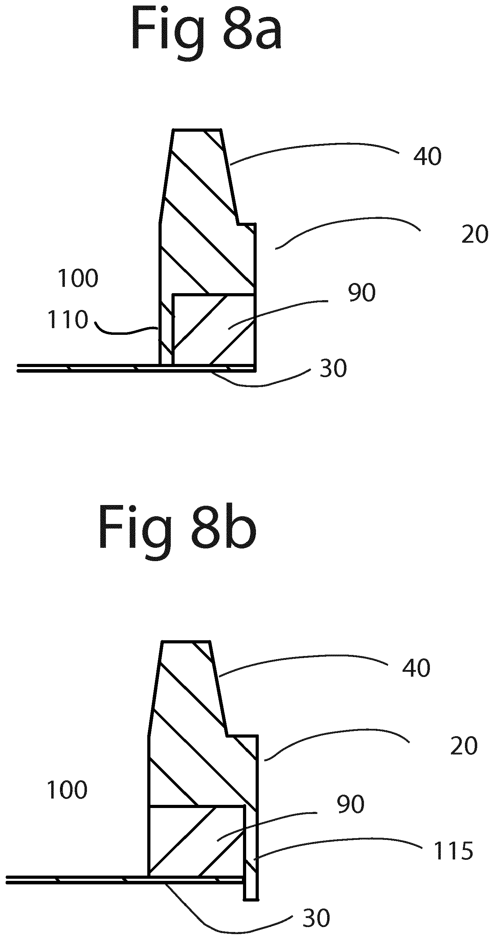

In Figur 8a ist eine Querschnittsansicht eines Bereichs einer weiteren beispielhaften Probenkammer gemäß der Erfindung gezeigt. Diese entspricht im Wesentlichen der Probenkammer gemäß der Ansicht aus Figur 5b. In diesem Fall ist das erste Element 40 jedoch so ausgebildet, dass ein Teil des ersten Elements 40 das zweite Element 90 zum Reservoir hin wenigstens teilweise abdeckt. Dafür ist ein Bereich 110 des ersten Elements 40 vorgesehen, der über die Grundfläche des ersten Elements 40 hinausragt. Durch diese Abdeckung des Elastomers durch den Bereich 110 kann das Kontaminierungsrisiko für eine Flüssigkeit, die im Reservoir 100 angeordnet wird, weiter verringert werden. In diesem Fall besteht das erste Element 40 vorzugsweise aus einem thermoplastischen Kunststoff, beispielsweise aus COC, COP, PE, PS, PC und/oder PMMA.

Die Höhe des Bereichs 110, also die Ausdehnung in einer Richtung senkrecht zur Kontaktfläche des ersten Bauteils, ist dabei bevorzugt kleiner als die Höhe des zweiten Elements 90, um eine sichere Verbindung zu gewährleisten.

In Figur 8b ist eine Querschnittsansicht eines Bereichs einer weiteren beispielhaften Probenkammer gezeigt. Diese entspricht im Wesentlichen der Probenkammer gemäß der Ansicht aus Figur 5b. In diesem Fall ist das erste Element 40 jedoch so ausgebildet, dass ein Teil des ersten Elements 40 das zweite Element 90 auf einer dem Reservoir 100 abgewandten Seite abdeckt. Dafür ist ein Bereich 115 des ersten Elements 40 vorgesehen, der über die Grundfläche des ersten Elements 40 hinausragt. Durch diese Abdeckung des Elastomers kann das Beschädigungsrisiko für das zweite Element 90 verringert werden. In diesem Fall besteht das erste Element 40 vorzugsweise aus einem thermoplastischen Kunststoff, beispielsweise aus COC, COP, PE, PS, PC und/oder PMMA.

Die Höhe des Bereichs 115, also die Ausdehnung in einer Richtung senkrecht zur Kontaktfläche des ersten Bauteils, ist dabei bevorzugt größer als die Höhe des zweiten Elements 90. Damit kann auch das zweite Bauteil 30 wenigstens teilweise vor möglichen Beschädigungen geschützt werden.

In den bisherigen Beispielen wurden zum Herstellen der Probenkammer zwei Bauteile 20 und 30 miteinander verbunden. Es kann jedoch auch wenigstens ein weiteres Bauteil vorgesehen sein. Beispielsweise kann ein drittes Bauteil bereitgestellt werden, das insbesondere mit dem ersten Bauteil 20 verbunden wird. Dazu kann ein weiterer Bereich des ersten Bauteils 20, in dem wenigstens ein Teil des Elastomers angeordnet ist, mit einem Plasma, mit Plasmaabgas und/oder mit einem reaktiven Gas behandelt werden. Wenigstens ein Teil dieses behandelten Bereichs kann dann mit dem dritten Bauteil verbunden werden.

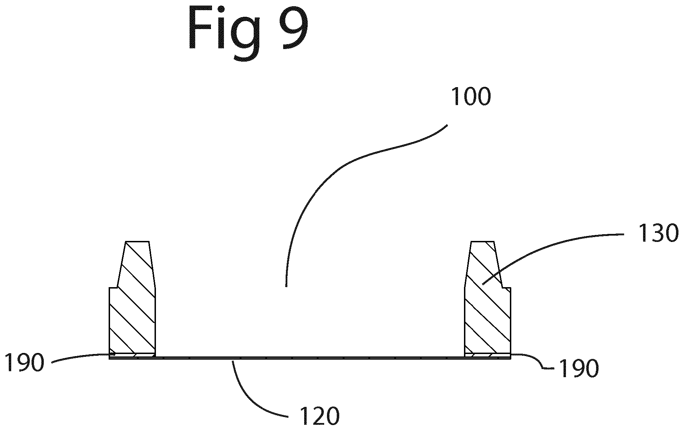

In den gezeigten Beispielen bildet das erste Bauteil 20 nach dem Verbinden eine Seitenwand und das zweite Bauteil 30 einen Boden eines Reservoirs 100. Es ist jedoch auch denkbar, dass das erste Bauteil 20 nach dem Verbinden einen Boden und das zweite Bauteil 30 eine Seitenwand eines Reservoirs bildet. Ein entsprechendes Beispiel ist in der Figur 9 dargestellt.

In Figur 9 ist eine beispielhafte Probenkammer gezeigt, umfassend ein erstes Bauteil 120, das ein Elastomer im Bereich 190 umfasst. Das Elastomer kann dabei auf einer Kunststoffplatte angeordnet sein. Das Elastomer im Kontaktbereich 190 wird dann mit einem Plasma, mit Plasmaabgas und/oder mit einem reaktiven Gas behandelt.

Das zweite Bauteil 130 kann aus Glas bestehen oder im Kontaktbereich mit dem Elastomer mit Glas beschichtet sein. Dieses zweite Bauteil 130 wird dann an das erste Bauteil 120 angepresst, wodurch eine feste Verbindung der Bauteile erreicht werden kann.

Die Geometrie der Reservoire oder der Probenkammern ist nicht auf die in den Figuren gezeigte Form beschränkt. Es sind auch beliebige andere Geometrien möglich, wie z.B. Kanäle die durch Verbinden eines ersten Bauteils mit entsprechenden Aussparungen mit einem zweiten Bauteil gebildet werden.

## Patentansprüche

1. Verfahren zum Herstellen einer Probenkammer umfassend die Schritte:
Bereitstellen eines ersten (20) und eines zweiten (30) Bauteils, wobei das erste Bauteil (20) ein Elastomer umfasst;
Behandeln des Elastomers im Bereich einer Kontaktfläche des ersten Bauteils (20) und/oder Behandeln einer Kontaktfläche des zweiten Bauteils (30) mit einem Plasma, mit Plasmaabgas und/oder mit einem reaktiven Gas; und
Verbinden der Kontaktfläche des ersten Bauteils (20) mit der Kontaktfläche des zweiten Bauteils (30);
wobei durch das Verbinden des ersten (20) und des zweiten (30) Bauteils ein Reservoir (100) erhalten wird, wobei das erste Bauteil (20) eine Seitenwand des Reservoirs (100) bildet, wobei das erste Bauteil ein erstes und ein damit verbundenes zweites Element aufweist, wobei nur das zweite Element das Elastomer umfasst, und wobei das erste Element (40) einen Bereich (110) umfasst, der das zweite Element (90) zum Reservoir (100) hin wenigstens teilweise abdeckt.

2. Verfahren nach Anspruch 1, wobei das erste Bauteil (20) so ausgebildet ist, dass ein Teil (50) des ersten Bauteils (20), in dem ein Teil des Elastomers angeordnet ist, nach dem Verbinden des ersten (20) mit dem zweiten (30) Bauteil über eine Seitenfläche des zweiten Bauteils (30) hinausragt.

3. Verfahren nach Anspruch 2, wobei der Teil (50) so ausgebildet ist, dass er sich nach dem Verbinden in eine Richtung senkrecht zur Kontaktfläche des ersten Bauteils (20) erstreckt, insbesondere so, dass eine Seitenfläche des zweiten Bauteils (30) wenigstens teilweise abgedeckt oder überlappt wird.

4. Verfahren nach Anspruch 2 oder 3, wobei der Teil (50) so ausgebildet ist, dass er nach dem Verbinden über eine der Kontaktfläche des zweiten Bauteils (30) abgewandte Seite des zweiten Bauteils (30) hinausragt.

5. Verfahren nach einem der vorangegangenen Ansprüche, wobei das erste Bauteil (20) außerdem einen thermoplastischen Kunststoff umfasst.

6. Verfahren nach Anspruch 1, wobei die maximale oder mittlere Ausdehnung des zweiten Elements (90) in einer Richtung senkrecht zur Kontaktfläche des ersten Bauteils (20) maximal 50 %, insbesondere von 1 % bis 20 %, der gesamten maximalen oder mittleren Ausdehnung des ersten Bauteils (20) senkrecht zur Kontaktfläche des ersten Bauteils (20) beträgt.

7. Verfahren nach einem der vorangegangenen Ansprüche, wobei die Dicke des ersten Bauteils in einem Bereich, der wenigstens einen Teil des Elastomers umfasst oder aus wenigstens einem Teil des Elastomers besteht, geringer ist als die mittlere Dicke des ersten Bauteils.

8. Verfahren nach einem der vorangegangenen Ansprüche, umfassend ein Bestrahlen der Kontaktfläche des zweiten Bauteils (30) mit UV-Licht.

9. Verfahren nach einem der vorangegangenen Ansprüche, wobei das zweite Bauteil (30) wenigstens im Bereich der Kontaktfläche des zweiten Bauteils (30), ein Glas umfasst.

10. Verfahren nach einem der vorangegangenen Ansprüche, wobei das Elastomer ein Silikon, insbesondere Polydimethylsiloxan, PDMS, umfasst.

11. Verfahren nach einem der vorangegangenen Ansprüche, wobei das Elastomer eine Shore-A Härte von mehr als 40, insbesondere von 60 bis 80, aufweist.

12. Probenkammer für Mikroskopuntersuchungen erhalten durch ein Verfahren nach einem der vorangegangenen Ansprüche.

## Claims

1. Method of manufacturing a sample chamber, comprising the steps of:
providing a first (20) and a second (30) component, the first component (20) comprising an elastomer;
treating the elastomer in the region of a contact surface of the first component (20) and/or treating a contact surface of the second component (30) with a plasma, with plasma waste gas, and/or with a reactive gas; and
connecting the contact surface of the first component (20) with the contact surface of the second component (30);
wherein a reservoir (100) is obtained by connecting the first (20) and the second (30) component, wherein the first component (20) forms a side wall of the reservoir (100), wherein the first component comprises a first element and a second element connected thereto, wherein only the second element comprises the elastomer, and wherein the first element (40) comprises a region (110) which at least partially covers the second element (90) towards the reservoir (100).

2. Method according to claim 1, wherein the first component (20) is designed such that a part (50) of the first component (20) in which a part of the elastomer is arranged projects beyond a side face of the second component (30) after the connection of the first (20) with the second (30) component.

3. Method according to claim 2, wherein the part (50) is designed such that, after the connection, it extends into a direction perpendicular to the contact surface of the first component (20), in particular in such a way that a side face of the second component (30) is at least partially covered or overlapped.

4. Method according to claim 2 or 3, wherein the part (50) is designed such that it projects, after the connection, beyond a side of the second component (30) facing away from the contact surface of the second component (30).

5. Method according to one of the preceding claims, wherein the first component (20) moreover comprises a thermoplastic.

6. Method according to claim 1, wherein the maximum or mean dimension of the second element (90) in a direction perpendicular to the contact surface of the first component (20) amounts to maximally 50 %, in particular 1 % to 20 %, of the total maximal or mean dimension of the first component (20) perpendicular to the contact surface of the first component (20).

7. Method according to one of the preceding claims, wherein the thickness of the first component is smaller in a region which comprises at least a part of the elastomer or consists of at least a part of the elastomer, than the mean thickness of the first component.

8. Method according to one of the preceding claims, comprising an irradiation of the contact surface of the second component (30) with UV light.

9. Method according to one of the preceding claims, wherein the second component (30) comprises, at least in the region of the contact surface of the second component (30), a glass.

10. Method according to one of the preceding claims, wherein the elastomer comprises a silicone, in particular polydimethylsiloxane, PDMS.

11. Method according to one of the preceding claims, wherein the elastomer has a Shore A hardness of more than 40, in particular of 60 to 80.

12. Sample chamber for microscopic examinations, obtained by a method according to one of the preceding claims.

## Revendications

1. Procédé de fabrication d'une chambre d'échantillon, comprenant les étapes suivantes :
fourniture et préparation d'une première (20) et d'une deuxième pièce (30), la première pièce (20) comportant un élastomère ;
traitement de l'élastomère dans la zone d'une surface de contact de la première pièce (20) et/ou traitement d'une surface de contact de la deuxième pièce (30) au moyen d'un plasma, au moyen d'émissions de gaz d'un plasma et/ou au moyen d'un gaz réactif ; et
liaison de la surface de contact de la première pièce (20) avec la surface de contact de la deuxième pièce (30) ;
procédé
d'après lequel la liaison de la première (20) et de la deuxième pièce (30) conduit à la formation d'un réservoir (100),
d'après lequel la première pièce (20) forme une paroi latérale du réservoir (100),
d'après lequel la première pièce comprend un premier élément et un deuxième élément qui y est relié,
d'après lequel seul le deuxième élément comporte l'élastomère,
et d'après lequel le premier élément (40) comporte une zone (110), qui recouvre au moins partiellement le deuxième élément (90) en direction du réservoir (100).

2. Procédé selon la revendication 1, d'après lequel la première pièce (20) est d'une configuration telle, qu'une partie (50) de la première pièce (20), dans laquelle est agencée une partie de l'élastomère, fait saillie au-delà d'une surface latérale de la deuxième pièce (30) après avoir réalisé la liaison entre la première (20) et la deuxième pièce (30).

3. Procédé selon la revendication 2, d'après lequel la partie (50) est d'une configuration telle qu'après la liaison, elle s'étende dans une direction perpendiculaire à la surface de contact de la première pièce (20), notamment de manière telle qu'une surface latérale de la deuxième pièce (30) soit au moins partiellement recouverte ou chevauchée.

4. Procédé selon la revendication 2 ou la revendication 3, d'après lequel la partie (50) est d'une configuration telle, qu'après la liaison, elle fasse saillie au-delà d'un côté de la deuxième pièce (30), qui est situé à l'opposé de la surface de contact de la deuxième pièce (30).

5. Procédé selon l'une des revendications précédentes, d'après lequel la première pièce (20) comprend en outre une matière plastique thermoplastique.

6. Procédé selon la revendication 1, d'après lequel l'étendue maximale ou moyenne du deuxième élément (90), dans une direction perpendiculaire à la surface de contact de la première pièce (20), vaut au maximum 50%, notamment de 1% à 20% de l'étendue maximale ou moyenne de la première pièce (20), perpendiculairement à la surface de contact de la première pièce (20).

7. Procédé selon l'une des revendications précédentes, d'après lequel l'épaisseur de la première pièce dans une zone qui comprend au moins une partie de l'élastomère ou est constituée par au moins une partie de l'élastomère, est inférieure à l'épaisseur moyenne de la première pièce.

8. Procédé selon l'une des revendications précédentes, comprenant une opération d'irradiation de la surface de contact de la deuxième pièce (30) avec de la lumière UV.

9. Procédé selon l'une des revendications précédentes, d'après lequel la deuxième pièce (30) comprend un verre, au moins dans la zone de la surface de contact de la deuxième pièce (30).

10. Procédé selon l'une des revendications précédentes, d'après lequel l'élastomère comprend un silicone, notamment un polydiméthylsiloxane, dit PDMS.

11. Procédé selon l'une des revendications précédentes, d'après lequel l'élastomère présente une dureté Shore-A de plus de 40, notamment de 60 à 80.

12. Chambre d'échantillon pour analyses au microscope, obtenue par un procédé selon l'une des revendications précédentes.
